# EUROPEAN PATENT APPLICATION

(11) **EP 3 205 712 A1**
(43) Date of publication of application: **16.08.2017**
(21) Application number: 15849728.9
(22) Date of filing: 05.10.2015
(51) Int. Cl.: C12M 1/34, C12M 1/00

(54) **CELL-CAPTURING DEVICE**

(30) Priority: 08.10.2014 JP 2014207218
(71) Applicant: Hitachi Chemical Co., Ltd., Chiyoda-ku Tokyo 100-6606 (JP)
(72) Inventor: KOTATO Akio, Tokyo 100-6606 (JP); KIKUHARA Yoshihito, Tokyo 100-6606 (JP); ODAGIRI Taihei, Tokyo 100-6606 (JP); KANETOMO Masafumi, Tokyo 168-0082 (JP)
(74) Representative: Hoffmann Eitle
(86) International application number: PCT/JP2015/078211
(87) International publication number: WO 2016/056512

(57) **Abstract**

A cell-capturing device includes: one or more introduction channels for introducing test liquid containing cells or treatment liquid for treating the cells in the test liquid into the inside thereof; a discharge channel for discharging the test liquid or the treatment liquid to the outside; a filter having a plurality of through-holes formed in a thickness direction, and being disposed in a channel between introduction channel and the discharge channel so that the test liquid or the treatment liquid passes through the through-holes; an introduction region formed between the filter and the introduction channel in a channel between the introduction channel and the discharge channel; a discharge region formed between the filter and the discharge channel; and a housing part accommodating at least a part of the introduction channel, the introduction region, and the discharge region therein, wherein the cell-capturing device further includes a pre-treatment part formed at a position apart from a connection part with the introduction region on at least one of the introduction channels, and formed by a spatial region having a diameter larger than that of the introduction channel.

## Description

### Technical Field

The present invention relates to a cell-capturing device for capturing specific cells that have entered test liquid through a filter.

### Background Art

Development of a cancer, which is also called a "malignant tumor," may cause a serious trouble to maintain life support of a living body, and thus a treatment thereof has been extensively studied. The presence or absence of metastasis of a cancer is used as a guideline when a treatment strategy of a cancer patient is decided. The metastasis is caused in a manner in which cancer cells enter into blood vessels or lymphatic vessels, through which the cancer cells are spread throughout the body, and they move into other organs. Important information can be obtained by the measurement of the presence or absence, or the amount of the cancer cells in the blood when the cancer metastasis is predicted. The cancer cells, which circulate through the human body via the blood vessels or lymphatic vessels, are called "Circulating Tumor Cell: (CTC)."

As a conventional technique to catch CTC for a metastasis cancer test or an anti-cancer evaluation, as shown, for example, in Patent Literature 1, a method for catching CTC through a filter is known. Patent Literature 1 shows a production method using a semi-conductor technique of a filter, a shape of a cell unit in which filters are stored, and a structure of a channel through which blood and treated liquid flow. Specifically, it shows a constitution in which blood containing cancer cells is poured into the cell unit in which the filters are stored to catch the cancer cells, and the cells on the filters are stained to identify the cancer cells. Patent Literatures 2 and 3 disclose a body fluid-treating system containing a cell-treating cartridge capable of providing at least one of a physical action, a chemical action and a physiological activation to at least one of cancer cells and immune cells by passing a cell dispersion containing at least one of the cancer cells and the immune cells through the cartridge. Patent Literature 4 discloses a micro fluid device containing a polydimethylsiloxane (PDMS) upper member having a sample inlet and a lower member having a sample outlet above and under a nickel substrate having fine through holes.

### Citation List

### Patent Literature

[Patent Literature 1] US-2011/0053152 A
[Patent Literature 2] Japanese Unexamined Patent Publication No. 2010-227011
[Patent Literature 3] Japanese Unexamined Patent Publication No. 2010-75191
[Patent Literature 4] Japanese Unexamined Patent Publication No. 2011-163830

### Summary of Invention

### Technical Problem

When the devices described in Patent Literatures 1 to 4 are used, however, if air is contained in the channels of the device, a treatment efficiency of the sample is remarkably reduced, and a measurement accuracy may be possibly reduced. In addition, foreign substances in the treatment liquid are accumulated on the filters, and may obstruct the cell capture.

In view of the circumstances described above, the present invention has been made, and an object of the present invention, in particular, is to provide a cell-capturing device, which suppress the entering of air to the vicinity of a filter capturing cells and, at the same time, removes foreign substances in liquid, whereby the capturing treatment of the cells can be reproducibly performed.

### Solution to Problem

In order to achieve the object described above, a cell-capturing device according to an embodiment of the present invention includes: one or more introduction channels for introducing test liquid containing cells or treatment liquid for treating the cells in the test liquid into the inside thereof; a discharge channel for discharging the test liquid or the treatment liquid to the outside; a filter having a plurality of through-holes formed in a thickness direction, and being disposed in a channel between introduction channel and the discharge channel so that the test liquid or the treatment liquid passes through the through-holes; an introduction region formed between the filter and the introduction channel in a channel between the introduction channel and the discharge channel; a discharge region formed between the filter and the discharge channel; and a housing part accommodating at least a part of the introduction channel, the introduction region, and the discharge region therein, wherein the cell-capturing device further includes a pre-treatment part formed at a position apart from a connection part with the introduction region on at least one of the introduction channels, and formed by a spatial region having a diameter larger than that of the introduction channel.

The pre-treatment part is provided in the introduction channel, and thus air bubbles contained in the treatment liquid or test liquid flowing in the introduction channel are separated, and are retained in the spatial region in the pre-treatment part. Here, the specific gravity of the air bubbles is smaller than that of the liquid, and thus the air bubbles separated move to the upper part of the liquid and retained there. The supply of the treatment liquid or test liquid containing the air bubbles toward the vicinity of the filter, disposed in the secondary stage from the pre-treatment part, is suppressed, and the treatment of capturing the cells can be reproducibly performed.

Here, according to an aspect, the pre-treatment part may contain a second filter having a plurality of through-holes formed in a thickness direction, and being disposed so that the test liquid or the treatment liquid passes through the through-holes.

When the pre-treatment part contains the second filter, the foreign substances in the liquid can be removed, and thus it is possible to reproducibly perform the treatment of capturing the cells through the filter. When the second filter is provided in the pre-treatment part, the removal effect of the air bubbles is improved and the accuracy of the treatment of capturing the cells is also improved.

Furthermore, according to an aspect, the channel between the spatial region and the introduction region may pass outside the housing part.

In this case, the complication of the inner structure of the housing part can be prevented compared to a structure in which the channel between the spatial region and the introduction region is provided in the housing part, and thus it is possible to reduce the production cost.

Furthermore, according to an aspect, the introduction region may be curved at an end part thereof. When the end part of the introduction region is made to have a curved shape, the retention of the air bubbles in the introduction region 51 can be further prevented.

### Advantageous Effects of Invention

According to the present invention, a cell-capturing device capable of reproducibly performing a treatment of capturing cells is provided.

### Brief Description of Drawings

Fig. 1 is a view illustrating a cell-capturing apparatus containing a cell-capturing device according to a first embodiment.
Fig. 2 is a perspective view showing the cell-capturing device according to the first embodiment.
Fig. 3 is a front view showing the cell-capturing device according to the first embodiment.
Fig. 4 is a top view showing the cell-capturing device according to the first embodiment.
Fig. 5 is an exploded perspective view showing the cell-capturing device according to the first embodiment.
Fig. 6 is an exploded perspective view showing the cell-capturing device according to the first embodiment.
Fig. 7 is a perspective view showing a cell-capturing device according to a second embodiment.
Fig. 8 is a front view showing the cell-capturing device according to the second embodiment.
Fig. 9 is a top view showing the cell-capturing device according to the second embodiment.
Fig. 10 is an exploded perspective view showing the cell-capturing device according to the second embodiment.
Fig. 11 is an exploded perspective view showing the cell-capturing device according to the second embodiment.
Fig. 12 is an exploded perspective view showing the cell-capturing device according to a modification of the second embodiment.
Fig. 13 is a front view showing a cell-capturing device according to a third embodiment.
Fig. 14 is a top view showing the cell-capturing device according to the third embodiment.
Fig. 15 is an exploded perspective view showing the cell-capturing device according to the third embodiment.
Fig. 16 is an exploded perspective view showing the cell-capturing device according to the third embodiment.

### Description of Embodiments

Referring to drawings attached, modes for carrying out the present invention are explained in detail below. In the explanation of the drawings, the same signs are applied to the same functions, and the overlapped explanations are omitted. In the instant specification, a numerical value range expressed by "from ... to..." refers to a range including the numbers described after "from" and after "to" as the minimum value and the maximum value.

### (Cell-Capturing Apparatus)

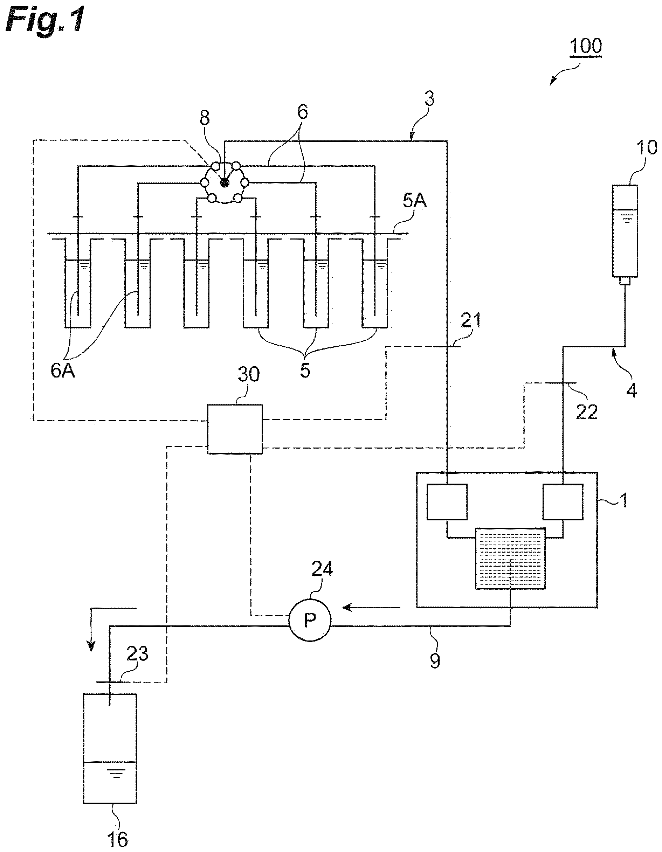

Fig. 1 is a view illustrating a structure of a cell-capturing apparatus containing a cell-capturing device according to a first embodiment. The cell-capturing apparatus is an apparatus for capturing cells contained in blood by filtering the blood, which is a test liquid, through a filter. In addition, the cell identification is performed and the number of cell bodies is counted by staining the cells captured through the filter with treatment liquid, such as staining liquid. In the following embodiment, a case in which test liquid is blood, and cells, which are targets to be captured, are circulating tumor cells (CTC) is explained, but the present invention is not limited thereto. For example, the test liquid may include blood, lymph, and the like, and a specific cell, which is the target to be captured, may include rare cells including CTC, circulating endothelial cells (CEC), circulating endothelial progenitor cells (CEP), and the like.

As shown in Fig. 1, a cell-capturing apparatus 100 is provided with a cell-capturing device 1 in which a filter for capturing cells is provided; a treatment liquid channel 3 for supplying treatment liquid (a reagent) to the cell-capturing device 1, formed of a flexible tube; and a test liquid channel 4 for supplying blood to the cell-capturing device 1, formed of a flexible tube. Multiple treatment liquid storage containers 5 containing treatment liquid (a reagent), different from each other, are provided on the upstream side of the treatment liquid channel 3. The treatment liquid, which are poured into the treatment liquid storage containers 5, may include staining liquid for staining the cells, washing liquid for washing the cells captured on the filter, fixing liquid for protecting the cells from modification, deterioration, and decomposition, permeation liquid for allowing the staining liquid to penetrate into the cell, and the like. The multiple treatment liquid storage containers 5, shown in Fig. 1, are sealed with a sealing member 5A, but the structure of apparatus is not particularly limited.

A hard tube 6A, connected to the flexible tube 6, is inserted into each of the multiple treatment liquid storage containers 5 to form an individual channel (a treatment liquid channel). These channels are connected to a selection valve 8, treatment liquid, which is connected to the treatment liquid channel 3, is selected by rotating the selection valve 8 and a hard tube 6A and a flexible tube 6, which are inserted into a treatment liquid storage container 5 in which the selected treatment liquid is accommodated, are connected to the treatment liquid channel 3. The structures of the hard tube 6A and the flexible tube 6 may be appropriately modified.

A blood storage container 10 (a test liquid storage container), in which blood containing specific cells are accommodated as the test liquid, is connected to the test liquid channel 4 connected to the cell-capturing device 1. According to the structure, both the treatment liquid and the blood are not supplied to the cell-capturing device 1 at the same time, but either of them is supplied. Which liquid of the treatment liquid or the blood is to be supplied is controlled by switching valves 21 and 22, which are connected to the treatment liquid channel 3 and the test liquid channel 4, respectively. For example, when the blood is supplied to the cell-capturing device 1, the valve 21 is closed and the valve 22 is opened. As the valves 21 and 22, a pinch valve, which shuts the flowing by compression deformation of the flexible tube, can be used.

When either the treatment liquid or the blood is supplied to the cell-capturing device 1, the supply of the liquid is performed by suction of the desired liquid using driving of a pump 24, provided on a discharge channel 9 formed of the flexible tube on the downstream side of the cell-capturing device 1. The pump 24 has a structure in which the flow rate of the liquid in the channel can be changed by changing the number of revolutions. As the pump 24, for example, a peristaltic pump, in which peristaltic points caused by the compression to the flexible tube are sequentially moved, can be used. The liquid such as the treatment liquid or the blood flows into the treatment liquid channel 3 or the test liquid channel 4 toward a direction of the cell-capturing device 1 by the driving of the pump 14, and is supplied in the cell-capturing device 1. The structure is such that the liquid, which has passed through the cell-capturing device 1, is poured into a waste liquid container 16 through the discharge channel 9. Owing to the structures described above, the cells in the blood are captured by the filter provided on the channel in the cell-capturing device 1 and, at the same time, the cells are stained by the staining liquid.

In the cell-capturing device 1, at least one pre-treatment part is provided in which a pre-treatment of the treatment liquid or the test liquid is performed. Specifically, a first pre-treatment part in which the treatment liquid is pre-treated is provided on a channel connected to the treatment liquid channel 3 which supplies the treatment liquid to the cell-capturing device 1, and a second pre-treatment part in which the test liquid is pre-treated is provided on a channel connected to the test liquid channel 4 which supplies the blood to the cell-capturing device 1. The details of the cell-capturing device 1 are described below.

The control of each part described above is performed by a control part 30 (selection tool). Specifically, the selection valve 8, the valves 21, 22 and 23, and the pump 24 are driven by instructions from the control part 30. The control part 30 contains program input functions for inputting a program capable of controlling starting and stopping of each part described above, and a driving mechanism, which successively actuates each device according to the program inputted in the control part, as described above, is added. A line through which the liquid flows is selected by the control part 30 (selection tool), and instructions concerning the switching of the valves and driving of the pump (liquid sending means) are sent to each part from the control part 30 based on the selection result.

### (Cell-Capturing Device)

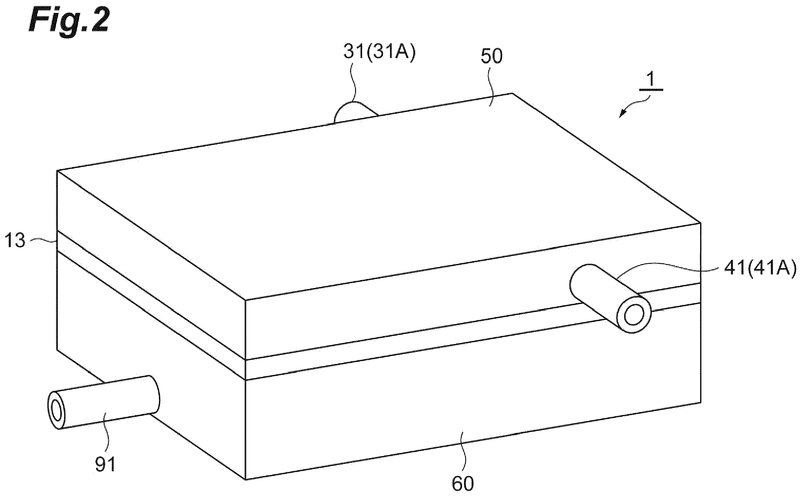

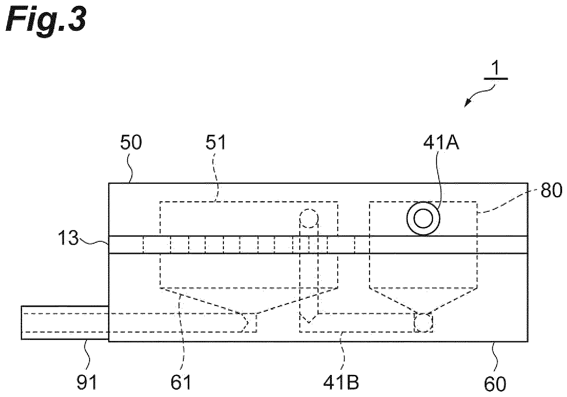

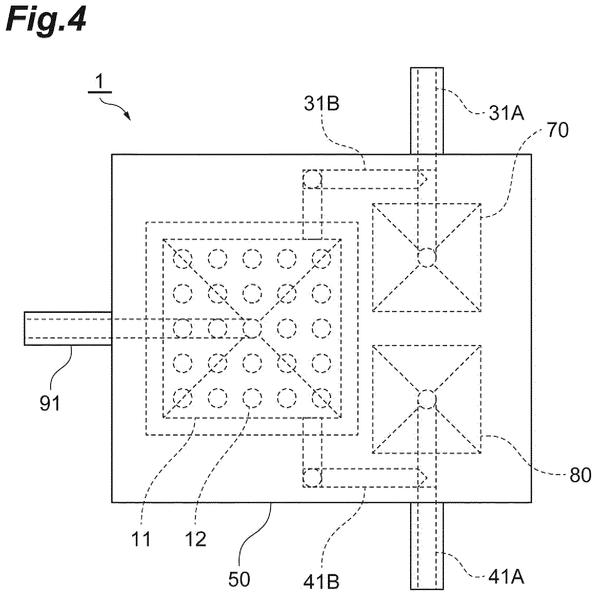

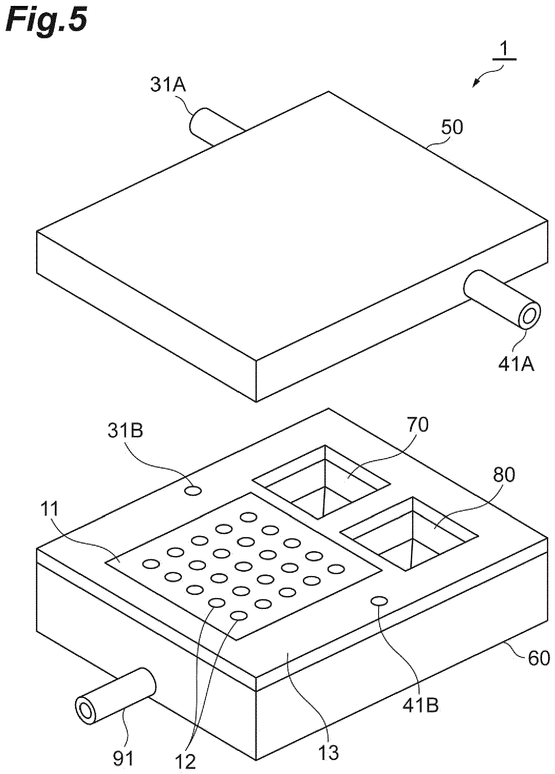

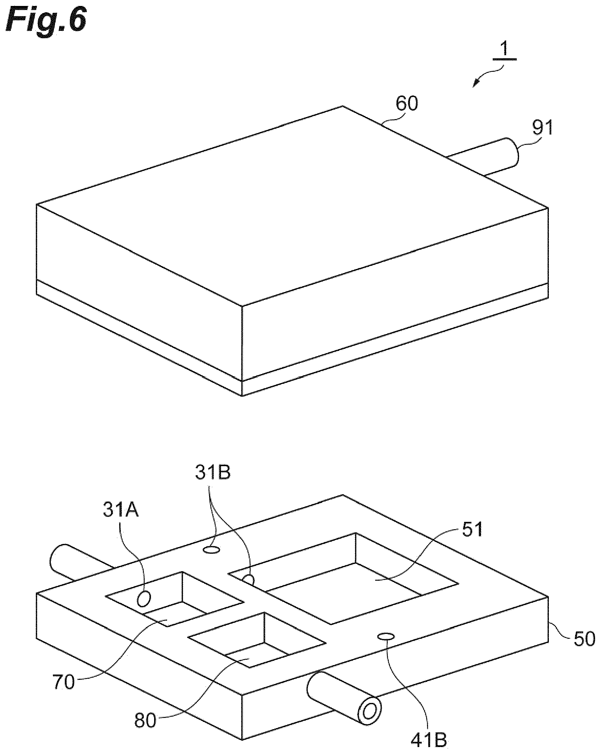

Next, the cell-capturing device 1 according to the first embodiment is explained using Fig. 2 to Fig. 6. Fig. 2 is an outside perspective view showing a cell-capturing device; Fig. 3 is a structural view showing the cell-capturing device when seen from the front; Fig. 4 is a structural view showing the cell-capturing device when seen from the top; and Fig. 5 and Fig. 6 are exploded perspective views showing the cell-capturing device.

The cell-capturing device 1 has a structure in which frame material 13 containing a filter 11 having multiple through-holes 12 is put between a lid member 50 and a storage member 60. The lid member 50 and the storage member 60 function as the housing part. The filter 11 is disposed in an interior space formed by combining the lid member 50 with the storage member 60. The filter 11 is, for example, formed of a metal, and has multiple through-holes 12 formed in the thickness direction thereof.

With respect to the size of the cell-capturing device 1, in terms of the workability and the observation ability, a length of one side of the lid member 50, when seen from a top view, is preferably from 10 mm to 100 mm, more preferably from 15 mm to 70 mm, even more preferably from 20 mm to 30 mm. The thickness is, in terms of the movable scope in a height direction of observation equipment, preferably from 2 mm to 20 mm, more preferably from 3 mm to 15 mm, even more preferably from 5 to 10 mm. When the cell-capturing device 1 is reinforced by clamping with a clip, the thickness described above does not include the thickness of the clip.

A metal material used for the filter 11 may include, but not limited to, gold, silver, copper, aluminum, tungsten, nickel, chromium, and alloys thereof. The metal may be used as it is, or may be used as an alloy with another metal or a metal oxide in order to provide functionality. It is preferable to use nickel, copper, gold, or a metal containing the same as a main component, in terms of cost and availability, and it is particularly preferable to use a metal containing nickel as a main component. When the filter 57 is formed from a material containing nickel as a main component, it is preferable that the surface of the nickel be plated with gold. As the oxidation of the filter surface can be prevented by the gold-plating, the adhesiveness to the specific cells, which is the target to be captured, (such as CTC) become uniform, and the reproducibility of data can be improved. If the conditions are met, a non-metal filter may be employed.

The filter 11 has preferably a thickness of from 3 µm to 100 µm. When the film thickness of the filter 11 is within the range described above, the filter can be easily handled, and is suitable for precision machining. It is preferable that a region where the through-holes 12 are formed in the filter 11 (which corresponds to a region through which the test liquid or treatment liquid can be passed) have an area of from 25 mm² to 1000 mm², more preferably from 25 mm² to 400 mm², even more preferably from 25 mm² to 250 mm². When the area of the region where the through-holes 12 are formed in the filter 11 is more than 1000 mm², dead spaces are increased. When the area of the region where the through-holes 12 are formed in the filter 11 is less than 25 mm², the treatment time is prolonged, and a problem of blocking of the filter may be likely to occur.

A shape of an opening of the through-holes in the filter 11 may include, but is not limited to, an oval, a circle, a rectangle, a square, a rounded rectangle, and a polygon. The rounded rectangle is a rectangle whose corners are rounded. One example of the rounded rectangle may be exemplified by a shape formed of 2 long sides whose lengths are the same and 2 semicircles, and a semicircle whose center is the middle point of a short side of the rectangle is formed outside the 2 short sides of the rectangle. The size of the cells to be captured depends on the kind of the cell, the hole diameter of the through-holes in the filter 57 can be appropriately selected according to the kind of the cell to be captured.

A material for the frame material 13 formed on the periphery of the filter 11 is not particularly limited so long as it can maintain airtightness when it is put between the lid member 50 and the storage member 60, and for example silicone rubber may be used. In order to maintain the airtightness, a caulking agent may be applied to the frame material 13.

In the cell-capturing device 1, an introduction channel 31 on the treatment liquid side, which is connected to the treatment liquid channel 3 formed of a flexible tube, and an introduction channel 41 on the test liquid side, which is connected to the test liquid channel 4 are formed. In the lid member 50 of the cell-capturing device 1, an introduction region 51, which is a space for leading the liquid to the through-holes 12 in the filter 11, communicating with the introduction channel 31 on the treatment liquid side and the introduction channel 41 on the test liquid side and being formed on the upper part of the filter 11. In the present embodiment, accordingly, the "introduction channel" refers to the introduction channel 31 on the treatment liquid side and the introduction channel 41 on the test liquid side, formed inside the cell-capturing device 1 among the channels. The "treatment liquid channel" and the "blood channel" refer to the treatment liquid channel 3 and the test liquid channel 4, which are connected to the upstream side of the introduction channel 31 on the treatment liquid side and the introduction channel 41 on the test liquid side, respectively, in the cell-capturing device 1.

In the cell-capturing device 1, a first pre-treatment part 70, in which the treatment liquid is pre-treated, is provided on the introduction channel 31 on the treatment liquid side, which introduces the treatment liquid. A second pre-treatment part 80, in which the test liquid is pre-treated, is provided on the introduction channel 41 on the test liquid side, which introduces the test liquid. In the following description, an introduction channel located at the primary stage from the first pre-treatment part 70 may sometimes be referred to as an introduction channel 31 A on the treatment liquid side, and an introduction channel located at the secondary stage from the first pre-treatment part 70 may sometimes be referred to as an introduction channel 31B on the treatment liquid side. In addition, an introduction channel located at the primary stage from the second pre-treatment part 80 may sometimes be referred to as an introduction channel 41 A on the test liquid side, and an introduction channel located at the secondary stage from the second pre-treatment part 80 may sometimes be referred to as an introduction channel 41B on the test liquid side.

In the storage member 60 of the cell-capturing device 1, a discharge region 61 is provided which is a space for discharging the liquid which has passed through the through-holes 12 in the filter 11 to the outside, being formed below the filter 11 and the depth at the center part thereof being deeper than that at the periphery. In the storage member 60, a discharge channel 91 for discharging the liquid in the discharge region 61 to the outside is provided which is communicated with the discharge region 61 and is connected to the discharge channel 9. The through-holes 12 formed in the filter 11, which is put between the lid member 50 and the storage member 60, have a diameter through which the cells to be captured cannot pass.

Mounting positions of the treatment liquid channel 3, the test liquid channel 4, and the discharge channel 9 on the cell-capturing device 1 are not limited to the positions shown in Fig. 2 to Fig. 6. It is not necessary, accordingly, that the treatment liquid channel 3 and the test liquid channel 4 are opposed to each other, and, for example, the treatment liquid channel 3 and the test liquid channel 4 may be connected on one side surface among the four side surfaces forming the cell-capturing device 1. The treatment liquid channel 3 and test liquid channel 4 may be connected to the top surface. Similarly, a mounting position of the discharge channel 9 may be appropriately changed. Mounting positions of the treatment liquid channel 3, the test liquid channel 4, and the discharge channel 9 are changed according to the introduction channel 31 on the treatment liquid side, the introduction channel 41 on the test liquid side, and the discharge channel 91 in the cell-capturing device 1, and thus arrangements of the introduction channel 31 on the treatment liquid side, the introduction channel 41 on the test liquid side, and the discharge channel 91 in the cell-capturing device 1 can be appropriately changed. The number of channels can also be appropriately changed.

The pre-treatment part, provided in the cell-capturing device 1, is explained in more detail. The pre-treatment part is provided for removing air bubbles in the liquid flowing in the channel. As described above, because the test liquid measured by the cell-capturing device 1 is blood, or the like, it is required to perform more accurately the measurement using a smaller amount of test liquid. In addition, when the air bubbles are contained in the test liquid or the treatment liquid, the accuracy of a volume, which is introduced into the device, is reduced. Further, when the air bubbles are retained on the filter, the introduction region 51 on the filter cannot be sufficiently utilized, and it may be likely to be difficult to preferably capture the cells by the filter 11. The cell-capturing device according to the present embodiment, accordingly, has a structure in which the pre-treatment parts are provided in the introduction channel 31 on the treatment liquid side and the introduction channel 41 on the test liquid side, whereby the bubbles are removed at the primary stage from the introduction region where the filter 11 is provided. The pre-treatment part is located at the primary stage from the introduction region where the filter 11 is provided, and is provided apart from the introduction region. The pre-treatment part and the introduction region are, accordingly, connected through a channel having a diameter smaller than the size of the regions. It is necessary to exchange the filter and the pre-treatment part per measurement of one test liquid. When the cell-capturing device 1 contains the filter 11 for capturing the cells, and pre-treatment parts for pre-treating the test liquid and the treatment liquid inside thereof, as described above, the work efficiency can be dramatically increased when multiple measurements of test liquid are continuously performed. In addition, the structure of the cell-capturing apparatus can be made more compact.

As shown in Fig. 3, Fig. 5, Fig. 6, and the like, in the cell-capturing device 1, the first pre-treatment part 70 is formed as a spatial region formed by the lid member 50 and the storage member 60. The first pre-treatment part 70 is characterized by having a diameter larger than that of the introduction channel 31 on the treatment liquid side. When the diameter of the introduction channel 31A on the treatment liquid side, which is the primary stage, is different from the diameter of the introduction channel 31B on the treatment liquid side, which is the secondary stage, the diameter of the first pre-treatment part 70 is set larger than that of the channel having the larger diameter. In order to retain the air bubbles in the first pre-treatment part 70 when the air bubbles are contained in the channel, the volume of the first pre-treatment part 70 is changed. The volume of the first pre-treatment part 70 is adjusted preferably to twice or more the volume of one air bubble, more preferably 5 times or more. Specifically, when the introduction channel 31A on the treatment liquid side has a diameter of 1 mm, the air bubble may have a length of about 1 mm to 3 mm in the channel. The first pre-treatment part 70 has, accordingly, a volume of, preferably, 10 mm³ or more.

It is preferable that, in the first pre-treatment part 70, the spatial region in which the air bubbles are retained be formed at an upper part of the introduction channel 31B on the treatment liquid side, which is an outlet of the treatment liquid. In the cell-capturing device 1, the upstream side, introduction channel 31 A on the treatment liquid side is provided at the upper part of the first pre-treatment part 70, and the treatment liquid is introduced from the introduction channel 31A on the treatment liquid side to the first pre-treatment part 70. The introduction channel 31B on the treatment liquid side is provided at a lower part of the first pre-treatment part 70. The treatment liquid moves, accordingly, from the upper part to the lower part in the first pre-treatment part 70. At that time, the first pre-treatment part 70 is provided at a position above an inlet to the introduction channel 31B on the treatment liquid side, and thus an inflow of the treatment liquid containing the air bubbles from the first pre-treatment part 70 to the introduction channel 31 B on the treatment liquid side is suppressed.

As shown in Fig. 3 and the like, the height of an introduction port to the introduction region 51 of the introduction channel 31 B on the treatment liquid side (an outlet of the introduction channel 31B on the treatment liquid side), and the height of an introduction port to the first pre-treatment part 70 of the introduction channel 31A on the treatment liquid side (an outlet of the introduction channel 31 A on the treatment liquid side) are set at almost the same. As a result, in the introduction channel 31B on the treatment liquid side, which is located at the secondary stage from the first pre-treatment part 70, the outlet provided on the lid member 50 side is set at a higher position than the position of the inlet provided at the storage member 60 side. Accordingly, the introduction channel 31B on the treatment liquid side is formed of an opening communicating the storage member 60, the frame material 13, and the lid member 50 (see Fig. 3). The method for forming the introduction channel 31B on the treatment liquid side is not particularly limited, and, for example, it may be produced by forming structures corresponding to the channels to a mold used for forming the lid member 50, the frame material 13, and the storage member 60. The channel may also be formed by forming the through-holes and then blocking a part of the through-holes with stoppers.

The second pre-treatment part 80 is provided in the same manner as in the first pre-treatment part 70. The second pre-treatment part 80 is formed as a spatial region formed by the lid member 50 and the storage member 60. An outlet of the upstream side, introduction channel 41 A on the test liquid side (an introduction port to the second pre-treatment part) is provided at the upper part of the second pre-treatment part 80, and the treatment liquid is introduced from the introduction channel 41A on the test liquid side to the second pre-treatment part 80. The introduction channel 41B on the test liquid side is provided at the lower part of the second pre-treatment part 80. The height of an introduction port to the introduction region 51 of the introduction channel 41B on the test liquid side (an outlet of the introduction channel 41B on the test liquid side), and the height of an introduction port to the second pre-treatment part 80 of the introduction channel 41 A on the test liquid side (an outlet of the introduction channel 41 A on the test liquid side) are set at almost the same. As a result, in the introduction channel 41B on the test liquid side, which is located at the secondary stage from the second pre-treatment part 80, the outlet provided on the lid member 50 side is at a higher position than the position of the inlet provided on the storage member 60 side. Accordingly, the introduction channel 41 B on the test liquid side is formed of an opening communicating the storage member 60, the frame material 13, and the lid member 50 (see Fig. 3).

The arrangement and the shape of the introduction channels 31 A and 31B on the treatment liquid side and the introduction channels 41 A and 41 B on the test liquid side are determined by the arrangement of the introduction region 51 for leading the liquid to the through-holes 12 in the filter 11, and the arrangements of the first pre-treatment part 70 and the second pre-treatment part 80, and thus they may be appropriately changed.

The cell-capturing device 1 is formed by overlapping the lid member 50 and the storage member 60 in a state in which the frame material 13 containing the filter 11 is put between them. At that time, the members may be positioned and then the positioned members may be fixed with clips from the outside, or the members may be fixed by thermocompression bonding. Then, the treatment liquid channel 3, the test liquid channel 4, and the discharge channel 9 are connected to the introduction channel 31 on the treatment liquid side, the introduction channel 41 on the test liquid side, and the discharge channel 91, respectively, thereby being attached to the cell-capturing apparatus 100.

In the cell-capturing apparatus 100 containing the cell-capturing device 1, the treatment liquid, supplied from the treatment liquid storage container 5, is supplied to the introduction region 51 of the filter 11 through the first pre-treatment part 70 in the cell-capturing device 1. At that time, the air bubbles in the treatment liquid are retained in the upper part of the first pre-treatment part 70, whereby the air bubbles are removed from the treatment liquid. The treatment liquid from which the air bubbles are removed is discharged from the discharge channel 91 to the outside of the cell-capturing device 1 through the introduction channel 31B on the treatment liquid side, the introduction region 51, the filter 11, and then the discharge region 61 in the cell-capturing device 1.

The blood supplied from the blood storage container 10 is supplied to the introduction region 51 of the filter 11 through the second pre-treatment part 80 in the cell-capturing device 1. At that time, the air bubbles in the treatment liquid are retained in the upper part of the second pre-treatment part 80, whereby the air bubbles are removed from the treatment liquid. The treatment liquid from which the air bubbles are removed is discharged from the discharge channel 91 to the outside of the cell-capturing device 1 through the introduction channel 41B on the test liquid side B, the introduction region 51, the filter 11, and then the discharge region 61 in the cell-capturing device 1.

It is preferable to use the cell-capturing device according to the embodiment of the present invention in a state in which the units and channels provided for connecting the units are previously filled with washing liquid, physiological saline or pure water.

Here, the cell-capturing device 1 according to the present embodiment contains the first pre-treatment part 70 on the introduction channel 31 on the treatment liquid side and, at the same time, the second pre-treatment part 80 on the introduction channel 41 on the test liquid side, whereby an internal pressure of the treatment liquid or the test liquid is temporarily decreased in the first pre-treatment part 70 or the second pre-treatment part 80 to separate the air bubbles contained in the treatment liquid flowing in the introduction channel 31 on the treatment liquid side or in the test liquid flowing in the introduction channel 41 on the test liquid side, thus resulting in the retention of the air bubbles in the spatial region of the pre-treatment part. Here, the specific gravity of the air bubble is smaller than that of the liquid, and thus the air bubbles separated move to the upper part of the liquid and are retained there. Consequently, the supply of the treatment liquid or test liquid containing the air bubbles toward the vicinity of the filter 11, disposed in the secondary stage from the first pre-treatment part 70 or the second pre-treatment part 80, is suppressed, and the treatment of capturing the cells can be reproducibly performed.

In the cell-capturing device 1, the first pre-treatment part 70 and the second pre-treatment part 80 are separately provided from the connection parts to the introduction region where the filter 11 is provided, whereby the flowing of the air bubbles separated to the introduction region 51 side is suppressed, and thus the treatment for capturing the cells can be reproducibly performed.

In addition, the cell-capturing device 1 has a structure in which introduction region 51 where the filter 11 is provided and the discharge region 61, the first pre-treatment part 70, and the second pre-treatment part 80 are accommodated in the same housing part (the lid member 50 and the storage member 60). In that case, the lengths of the introduction channel 31 on the treatment liquid side and the introduction channel 41 on the test liquid side can be made shorter. When the treatment for capturing the cells is performed using the cell-capturing device 1, it is necessary to perform the treatment after the all channels are filled with the test liquid or treatment liquid, in terms of the removal of the air bubbles. It can be considered, accordingly, if the device has a structure in which the channels are long, then the amount of liquid necessary for the measurement is increased. Meanwhile, in the cell-capturing device 1 in which the pre-treatment parts are integrated, the prolonged channels can be prevented by providing the pre-treatment part. In addition, the increase of the number of parts can be prevented even if the pre-treatment parts are provided, thus resulting in the improved handling. Further, comparing the case in which the pre-treatment parts are formed as separated bodies, the cost for providing the pre-treatment parts can be suppressed.

In a case in which the removal of the air bubbles on the filter 11 is further ensured, it is preferable that end parts (boundaries between the side surfaces and boundaries between the side surface and the top surface) in the introduction region 51 on the upper part of the filter be curved. When the sites have corners, the air bubbles may possibly be retained. Accordingly, the retention of the air bubbles in the introduction region 51 can be further prevented by making the end parts on the upper parts of the introduction region 51 to have the curved shape.

### (Second Embodiment)

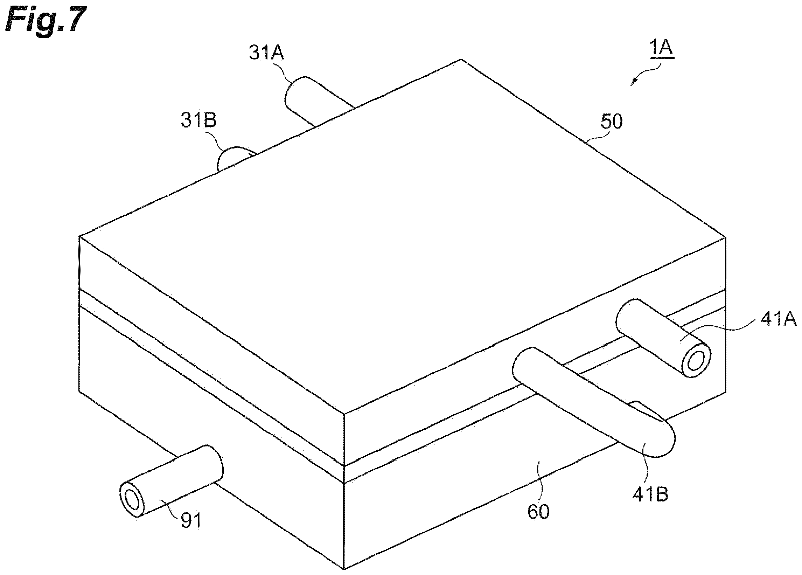

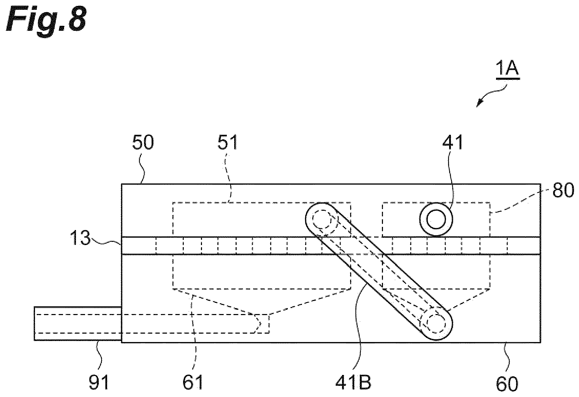

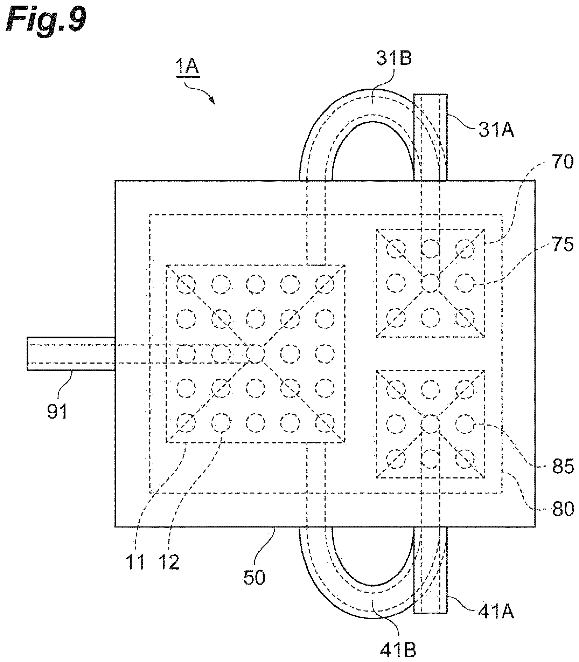

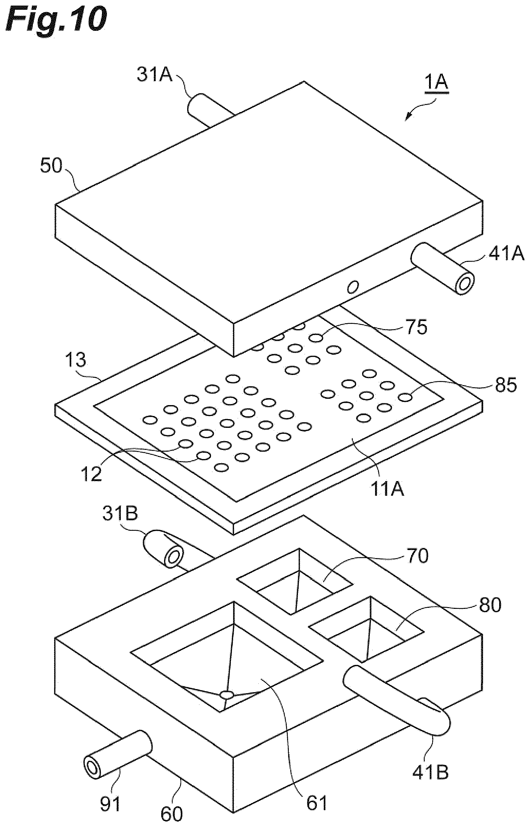

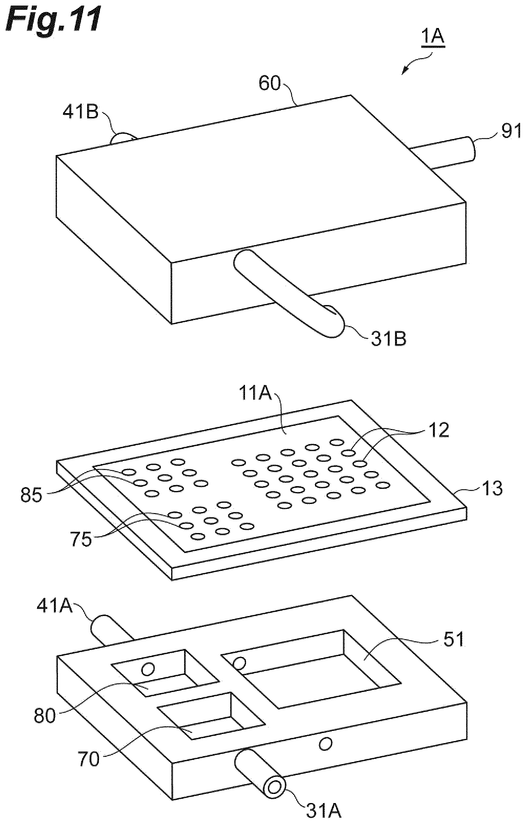

Next, a cell-capturing device according to a second embodiment is explained referring to Fig. 7 to Fig. 11. Fig. 7 is an outer perspective view showing the cell-capturing device; Fig. 8 is a structural view showing the cell-capturing device when seen from the front; Fig. 9 is a structural view showing the cell-capturing device when seen from the top; and Fig. 10 and Fig. 11 are exploded perspective views showing the cell-capturing device.

The cell-capturing device 1A according to the second embodiment is different from the cell-capturing device according to the first embodiment in the following points. The different points from the cell-capturing device 1 according to the first embodiment are that an introduction channel 31B on the treatment liquid side between a first pre-treatment part 70 and an introduction region 51, and an introduction channel 41B on the test liquid side B between a second pre-treatment part 80 and an introduction region 51 are attached so that they are protruded outward from a lid member 50 and a storage member 60, which form a housing part; and that a filter for removing foreign substances (a second filter) are provided in the first pre-treatment part 70 and the second pre-treatment part 80.

In the cell-capturing device according to the first embodiment, the channels are provided inside the housing part, and thus the more compact shape can be realized. On the other hand, because the internal channel structure may possibly be complicated, and thus it can be considered that the production cost is increased. In the cell-capturing device according to the second embodiment, the introduction channel 31B on the treatment liquid side and the introduction channel 41 B on the test liquid side are configured so that they go through outside the housing part, whereby the reduction of the production cost of the housing part is attempted. In addition, the introduction channel 31B on the treatment liquid side and the introduction channel 41 B on the test liquid side B are removable because they are configured so that they go through outward, and thus the operability can be improved.

As shown in Fig. 7 to Fig. 11, in the cell-capturing device 1A, the introduction channel 31B on the treatment liquid side and the introduction channel 41 B on the test liquid side are protruded outward from the lid member 50 and the storage member 60. Mounting positions of the introduction channel 31B on the treatment liquid side and the introduction channel 41 B on the test liquid side (connection positions to the first pre-treatment part 70, the second pre-treatment part 80, and the introduction region 51) are the same as in the cell-capturing device 1. In that case, the introduction channel 31B on the treatment liquid side and the introduction channel 41 B on the test liquid side may be formed of, for example, a silicone tube or a metal tube, or the like. Even if the introduction channel 31 B on the treatment liquid side and the introduction channel 41 B on the test liquid side are attached so that they are attached so that they are protruded outward as described above, the supply of the treatment liquid or the test liquid containing the air bubbles to the vicinity of the filter 11, disposed at the secondary stage from the first pre-treatment part 70 or the second pre-treatment part 80, can be suppressed, and the treatment for capturing the cells can be reproducibly performed.

When the introduction channel 31B on the treatment liquid side and the introduction channel 41 B on the test liquid side are formed of a flexible material such as a silicone tube, for example, a valve mechanism, which presses the introduction channel 31B on the treatment liquid and the introduction channel 41 B on the test liquid side B from the outside, may be provided. In addition, a configuration capable of removing the introduction channel 31 B on the treatment liquid side and the introduction channel 41 B on the test liquid side B, which are protruded outward, from the housing part formed of the lid member 50 and the storage member 60 may be made. In that case, it is possible to adopt a configuration in which only the housing part containing the filter 11 is removed from the measurement apparatus, and the introduction channel 31B on the treatment liquid side and the introduction channel 41 B on the test liquid side are attached to the apparatus.

A configuration in which either the introduction channel 31 B on the treatment liquid side or the introduction channel 41 B on the test liquid side is protruded outward may be adopted. This point may be appropriately changed. For example, it is possible to provide the introduction channel 31B on the treatment liquid side outside the cell-capturing device 1; that is, it is possible to provide a hydraulic circuit equivalent to the introduction channel 31 B on the treatment liquid side on the apparatus side to which the cell-capturing device 1 is attached. When this system is adopted, the shape of the cell-capturing device 1 can be simplified. Further, if necessary, this channel can be controlled.

In the cell-capturing device 1A, the first pre-treatment part 70 and the second pre-treatment part 80 are provided with filters 75 and 85 for removing foreign substances, respectively. Multiple through-holes, which are formed in a thickness direction of the filter 85 for removing the foreign substance and are disposed so that the test liquid can be passed through it, have preferably a hole diameter which is sufficiently larger than the hole diameter of the filter 11 and is incapable of capturing the cells to be captured. In that case, only the foreign substances in the test liquid can be preferably removed, and the capture of the cells, which are targets to be captured, in error can be prevented. With respect to the filter 75 for removing the foreign substance, which is disposed so that the treatment liquid is passed through the multiple through-holes, the hole diameter is not particularly limited so long as the foreign substances in the treatment liquid can be removed, but it is preferable to previously remove foreign substances whose size disturbs the capture of the cells. It is preferable, accordingly, that the hole diameter is equal to or smaller than the hole diameter of the cell-capturing filter.

The metal material used for the foreign substance-removing filters 75 and 85 may include, but not limited to, gold, silver, copper, aluminum, tungsten, nickel, chromium, alloys of these metals, and the like. The metal may be used as it is, or may be used as an alloy with another metal or a metal oxide in order to provide a function. It is preferable to use nickel, copper, gold, or a metal containing the same as a main component, in terms of cost and availability, and it is particularly preferable to use a metal containing nickel as a main component. When the foreign substance-removing filters 75 and 85 are formed from a material containing nickel as a main component, it is preferable that the surface of the nickel be plated with gold. As the oxidation of the filter surface can be prevented by the gold-plating, the foreign substance-removing performance can be highly maintained. It is also preferable that the filter surface be coated with a polymer harmless to the cells. In that case, however, the adhesiveness to the cells cannot be obtained.

When the first pre-treatment part 70 and the second pre-treatment part 80 are provided on the foreign substance-removing filters 75 and 85, respectively, the foreign substances can be removed in the first pre-treatment part 70 and the second pre-treatment part 80, and thus the treatment for capturing the cells on the filter 11 can be reproducibly performed. When the foreign substance-removing filters 75 and 85 are provided in the first pre-treatment part 70 and the second pre-treatment part 80, the effect of removing the air bubbles is improved in the first pre-treatment part 70 and the second pre-treatment part 80. This can be considered that when the treatment liquid or the test liquid is passed through the foreign substance-removing filters 75 and 85, the separation of the air bubbles is promoted by the foreign substance-removing filters 75 and 85. When there are the foreign substance-removing filters 75 and 85, accordingly, the air bubble-removing effect is improved and the accuracy of the treatment for capturing the cells is also improved.

In the cell-capturing device 1 according to the second embodiment, the filter 11 and the foreign substance-removing filters 75 and 85 are formed of one sheet 11A, and a frame material 13 is provided on the periphery of the sheet 11A (see Fig. 10). What structure of the filter 11, the foreign substance-removing filters 75 and 85 are adopted may be appropriately changed, as described above. When they are formed of the one sheet 11A, an elastic member may further be disposed so that the first pre-treatment part 70, the second pre-treatment part 80, the introduction region 51, and the discharge region 61 are not connected to each other on the upper surface side or under surface side of the sheet 11A. In addition, the foreign substance-removing filter may be disposed only on either first pre-treatment part 70 or the second pre-treatment part 80.

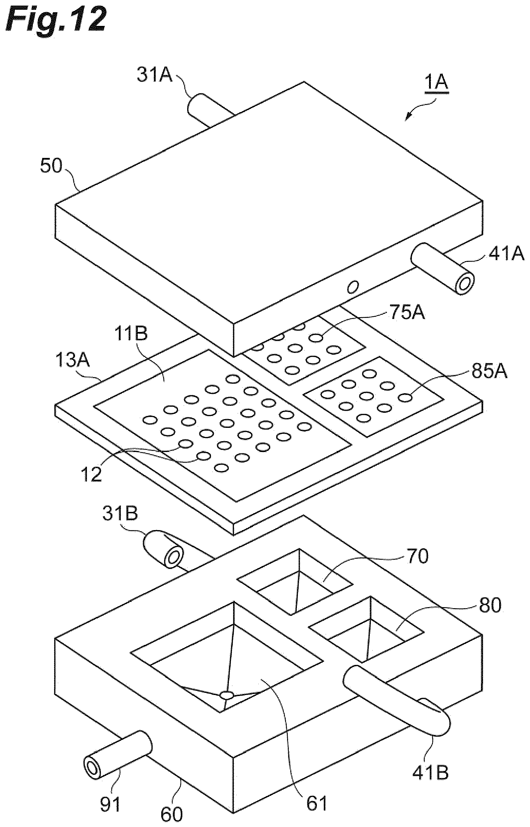

Fig. 12 is a view showing a structural modification of the filter part in the cell-capturing device 1. In Fig. 12, the filter 11B having the through-holes 12, and the foreign substance-removing filters 75A and 85A are provided to the frame materials 13A provided with openings for the filter at three positions. As described above, the structures of the filter and the foreign substance-removing filter can be variously modified.

### (Third Embodiment)

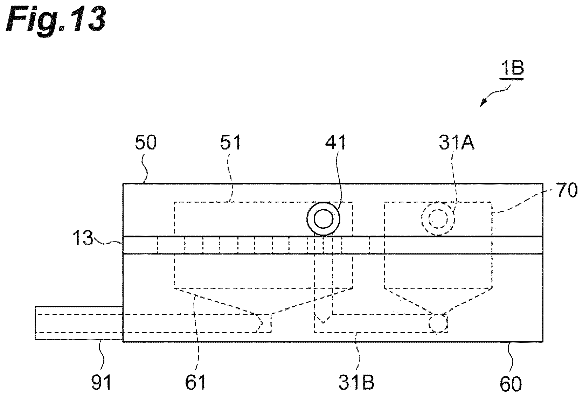

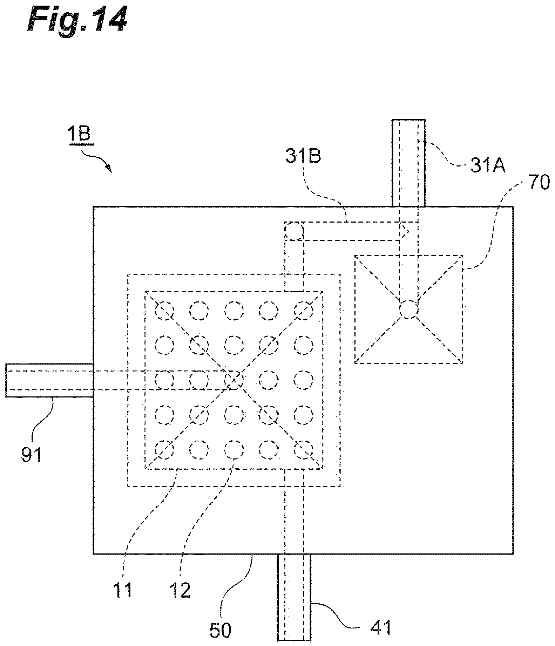

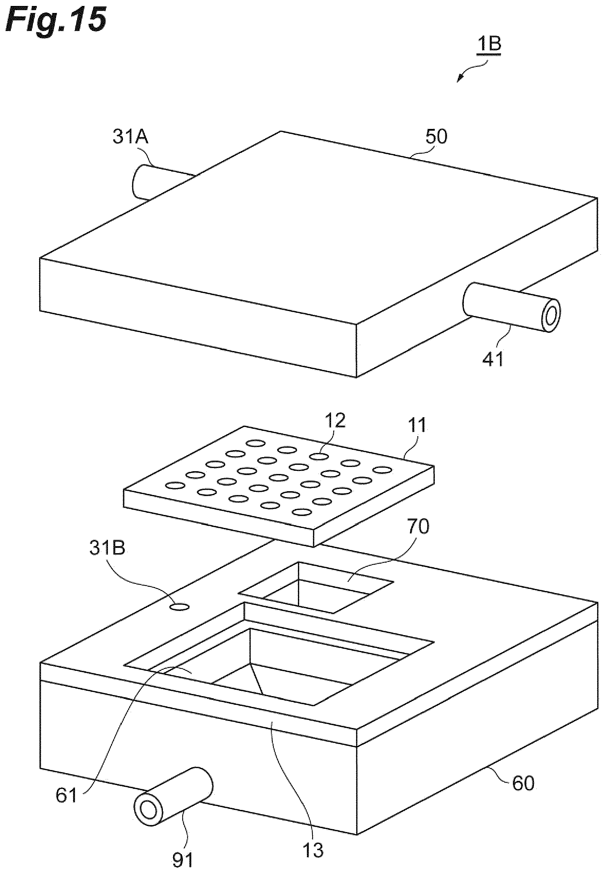

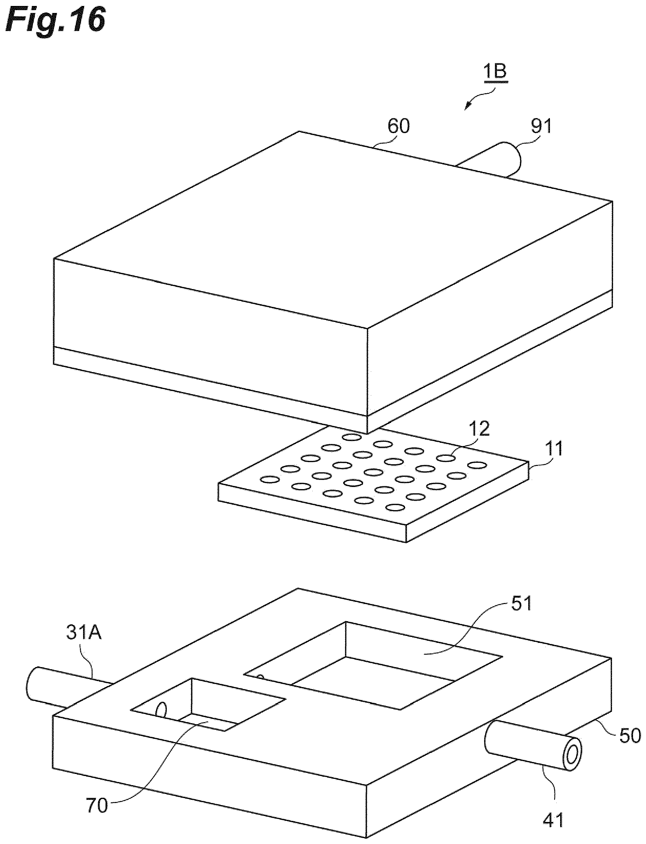

Next, a cell-capturing device according to a third embodiment is explained referring to Fig. 13 to Fig. 16. Fig. 13 is a structural view showing the cell-capturing device when seen from the front; Fig. 14 is a structural view showing the cell-capturing device when seen from the top; and Fig. 15 and Fig. 16 are exploded perspective views showing the cell-capturing device.

The cell-capturing device 1B according to the third embodiment is different from the cell-capturing device according to the first embodiment in the following point. The different point from the cell-capturing device 1 according to the first embodiment is that the cell-capturing device 1B does not contain a second pre-treatment part 80.

In the cell-capturing device 1 B, the introduction channel 41 on the test liquid side is directly connected to the introduction region 51 without passing through the second pre-treatment part. For that structure, the test liquid is introduced from the test liquid channel 4 to the introduction region 51 without going through the second pre-treatment part, and the like.

For example, it can be considered that there is a case in which the pre-treatment is not required for the test liquid, because the air bubbles are thoroughly removed. In that case, when the configuration in which the second pre-treatment part is not provided as the cell-capturing device 1 B is adopted, the channel can be shortened, and the amount of the test liquid used can be decreased. As described above, in the cell-capturing device having the multiple introduction channels, the pre-treatment part may be provided only on a part of the introduction channels. Even in such a case, the air bubbles can be removed from the liquid passing through the pre-treatment part, and thus the reproducibility of the capture of the cells can be improved.

The embodiments of the present invention have been explained above, but the cell-capturing device according to the present invention is not limited to the embodiments described above, and various changes can be made.

For example, in the embodiments described above, the case in which the cell-capturing device 1 is formed of the lid member 50 and the storage member 60 has been explained, and the shape of the housing part formed of the lid member 50 and the storage member 60 may be appropriately changed. The number of the introduction channels is not particularly limited so long as it is 1 or more.

In addition, a configuration in which valves are further provided on the introduction channel on the treatment liquid side, introduction channel on the test liquid side, and the discharge channel cell-capturing device 1, whereby the flow of the liquid in the device is controlled may be adopted.

### Reference Signs List

1 ... cell-capturing device, 5 ... treatment liquid storage container, 10 ... blood storage container (test liquid storage container), 11 ... filter, 31 ... introduction channel on treatment liquid side, 41 ... introduction channel on test liquid side, 50 ... lid member, 51 ... introduction region, 60 ... storage member, 61 ... discharge region, 70 ... first pre-treatment part, 80 ... second pre-treatment part, 100 ... cell-capturing apparatus.

## Claims

1. A cell-capturing device comprising:
one or more introduction channels for introducing test liquid containing cells or treatment liquid for treating the cells in the test liquid into the inside thereof;
a discharge channel for discharging the test liquid or the treatment liquid to the outside;
a filter having a plurality of through-holes formed in a thickness direction, and being disposed in a channel between introduction channel and the discharge channel so that the test liquid or the treatment liquid passes through the through-holes;
an introduction region formed between the filter and the introduction channel in a channel between the introduction channel and the discharge channel;
a discharge region formed between the filter and the discharge channel; and
a housing part accommodating at least a part of the introduction channel, the introduction region, and the discharge region therein, wherein
the cell-capturing device further comprises a pre-treatment part formed at a position apart from a connection part with the introduction region on at least one of the introduction channels, and formed by a spatial region having a diameter larger than that of the introduction channel.

2. The cell-capturing device according to claim 1, wherein the pre-treatment part contains a second filter having a plurality of through-holes formed in a thickness direction, and being disposed so that the test liquid or the treatment liquid passes through the through-holes.

3. The cell-capturing device according to claim 1 or 2, wherein the channel between the spatial region and the introduction region passes outside the housing part.

4. The cell-capturing device according to any one of claims 1 to 3, wherein the introduction region is curved at an end part thereof.
